# EUROPEAN PATENT SPECIFICATION

(11) **EP 3 530 279 B1**
(45) Date of publication and mention of the grant of the patent: **22.09.2021**
(21) Application number: 17865481.0
(22) Date of filing: 23.10.2017
(51) Int. Cl.: A61K 38/08, A61K 9/08, A61K 9/10, A61P 15/00

(54) **PHARMACEUTICAL COMPOSITION FOR USE IN TREATING FEMALE SEXUAL DYSFUNCTIONS**
PHARMAZEUTISCHE ZUSAMMENSETZUNGEN ZUR VERWENDUNG IN DER BEHANDLUNG VON WEIBLICHEN SEXUELLEN DYSFUNKTIONEN
COMPOSITION PHARMACEUTIQUE POUR L'UTILISATION DANS LE TRAITEMENT DE DYSFONCTIONS SEXUELLES FÉMININES

(30) Priority: 24.10.2016 RU 2016112341
(43) Date of publication of application: 28.08.2019
(73) Proprietor: OVB (Ireland) Limited, Dublin 2, D02 AT22 (IE)
(72) Inventor: MYASOEDOV, Nikolai Fedorovich, Moscow 125367 (RU); ANDREEVA, Lyudmila Alexandrovna, Moscow 123423 (RU); GOLIKOV, Dmitry Viktorovich, Moscow 127018 (RU); LOMONOSOV, Mikhail Yurievich, Moscow 115487 (RU)
(74) Representative: Cabinet Becker et Associés
(86) International application number: PCT/RU2017/050112
(87) International publication number: WO 2018/080353

(56) References cited:
- WO-A2-2013/151467
- CN-A- 104 274 818
- EA-B1- 013 948
- JP-A- 2015 515 855
- RU-C1- 2 130 030
- RU-C1- 2 318 533
- RU-C1- 2 404 793
- UA-U- 28 397
- PAVLOV TS: "Protivoyazvennyye effekty selanka i yego fragmentov. [Anti-ulcer effects of Selanka and its fragments]", Avtoreferat dissertatsii na soiskaniye uchonoy stepeni biologicheskikh nauk. [Abstract of dissertation for the degree of biological sciences], 2006, pages 1-23, XP009514517, Moskou, Russia

## Description

### Field of the Invention

The invention relates to the fields of medicine and pharmacology and to the treatment and/or prevention of female sexual dysfunction with a highly effective medication comprising a pentapeptide.

### Prior Art

Female sexual dysfunction (FSD) is one of various disorders of sexual function leading to a loss of interest in sexual activity, repeated failure to reach or maintain sexual arousal, impossibility to reach an orgasm following sufficient arousal. According to the International Classification of Diseases (ICD), there are the following types of sexual dysfunction that are not caused by organic disorders or diseases: lack or loss of sex drive, sexual aversion and lack of sexual pleasure, orgasmic dysfunction, vaginismus of non-organic origin, dyspareunia of non-organic origin, and other sexual dysfunctions not caused by organic disorders or diseases.

The need to develop a drug for treating female sexual dysfunction stems from a large number of women experiencing sexual dysfunction (Edward O. Laumann, Anthony Paik, Dale B. Glasser et all. A Cross-National Study of Subjective Sexual Well-Being Among Older Women and Men: Findings from the Global Study of Sexual Attitudes and Behaviors. Archives of Sexual Behavior, Vol. 35, No. 2, 2006, p. 145-161) caused by increased stress of modern life created by man-made and natural disasters, environmental degradation, urbanization, and political and economic instability in the modern world, the general trend towards aging of our society and insufficient effective tools and methods for treating female sexual dysfunctions available in medicine today.

Many different methods of treating female sexual dysfunction were proposed and used with various degrees of success. Said treatment methods were either only partially successful or had side effects making them unacceptable. The most effective preparation for clinical application today is Flibanserin. Still, there is a need to develop novel preparations for treating female sexual dysfunction.

Therapeutic peptides are widely used in medical practice. RU2507212, for example, discloses a group of peptides that exhibit activity stimulating sexual function and sexual activity:
A-Thr-Lys-Pro-B-C-D-X,
where A - 0, Met, Met(O), Thr, Ala, His, Phe, Lys, Gly
B - 0, Gly, Asp, Trp, Gin, Asn, Tyr, Pro, Arg
C - 0, Arg, Phe, Tyr, Gly, His, Pro, Lys
D - 0, Val, Gly, Tyr, Trp, Phe, His
X - OH, OCH₃, NH₂, where 0 - no amino acid residue on the condition that if
A ≠ 0, then B and/or C, and/or D ≠ 0, if B ≠ 0, then C and/or D ≠ 0, excluding tetrapeptides, and also peptides Phe-Thr-Lys-Pro-Gly, Thr-Lys-Pro-Pro-Arg, Thr-Lys-Pro-Arg-Gly.

Synthesized peptides of general formula A-Thr-Lys-Pro-B-C-D-X, as was established, can be recommended as stimulants of sexual function and sexual activity.

An acceptable dosage form for said peptides as well as therapeutically effective doses as well as an administration regimen, however, have not yet been developed. Most medications are known to reach a consumer as a finished product or a so-called pharmaceutical preparation. A finished pharmaceutical preparation is a composition containing the medication itself and excipients in a certain form or as an aggregate. A dosage form often not only determines the ease of administration, but also the other characteristics of the drug's efficacy, such as bioavailability, duration of action, etc.

### Summary of the Invention

The objective of the present invention is to develop and create an effective and safe medication for treating and/or preventing female sexual dysfunction, which could be used in clinical practice.

The technical result is the development and preparation of a stable and effective pharmaceutical preparation containing a peptide that exhibits activity stimulating sexual functions and sexual activity in a therapeutically effective dose, as well as developing an effective and safe composition for use in a method of treating female sexual dysfunction, comprising the use of the composition according to the invention.

Said technical result is achieved by way of development and preparation of a pharmaceutical composition for intranasal administration for use in preventing or treating female sexual dysfunction, HSDD, FSAD, or FSIAD, in a liquid dosage form comprising pentapeptide Thr-Lys-Pro-Arg-Pro or a pharmaceutically acceptable salt thereof at a 2 - 20g/L concentration, and at least one pharmaceutically acceptable excipient.

In particular embodiments, the pharmaceutical composition according to the invention additionally comprises a preservative.

In particular embodiments, the pharmaceutical composition comprises a therapeutically effective dose of pentapeptide Thr-Lys-Pro-Arg-Pro or a pharmaceutically acceptable salt thereof and a preservative at the following concentrations, g/L:

| | |
|---|---|
| Pentapeptide Thr-Lys-Pro-Arg-Pro or a pharmaceutically acceptable salt thereof | 2 - 20; |
| preservative | 0.095 - 0.105, |
| water | remaining |

In some embodiments, the pharmaceutically acceptable excipient is a carrier and/or a solvent.

In some embodiments, the preservative is benzalkonium chloride.

In particular embodiments, the pharmaceutically acceptable salt of pentapeptide Thr-Lys-Pro-Arg-Pro is acetate, hydrochloride, phosphate, sulfate, mesylate, or tosylate.

In particular embodiments, the liquid dosage form is a solution.

In some particular embodiments, the liquid dosage form is a spray.

In some particular embodiments, the therapeutically effective daily dose of pentapeptide Thr-Lys-Pro-Arg-Pro or a pharmaceutically acceptable salt thereof is 840 mg.

In some particular embodiments, the therapeutically effective daily dose of pentapeptide Thr-Lys-Pro-Arg-Pro or a pharmaceutically acceptable salt thereof is 1,680 mg.

In some particular embodiments, the therapeutically effective daily dose of pentapeptide Thr-Lys-Pro-Arg-Pro or a pharmaceutically acceptable salt thereof is 2,520 mg.

In some particular embodiments, the therapeutically effective daily dose of pentapeptide Thr-Lys-Pro-Arg-Pro or a pharmaceutically acceptable salt thereof is 3,360 mg.

In some particular embodiments, the therapeutically effective daily dose of pentapeptide Thr-Lys-Pro- Arg-Pro or a pharmaceutically acceptable salt thereof is 4,200 mg.

In some particular embodiments, the therapeutically effective daily dose of pentapeptide Thr-Lys-Pro-Arg-Pro or a pharmaceutically acceptable salt thereof is 5,040 mg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 800 - 5,100 mcg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 840 mcg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 1,680 mcg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 2,520 mcg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 3,360 mcg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 4,200 mcg.

In some embodiments, the pharmaceutical composition comprising pentapeptide Thr-Lys-Pro-Arg-Pro or a pharmaceutically acceptable salt thereof is administered to a patient in a daily dose of pentapeptide Thr-Lys-Pro-Arg-Pro or a pharmaceutically acceptable salt thereof equal to 5,040 mcg.

In some embodiments, the pharmaceutical composition is administered daily for 1-31 days.

In some embodiments, the pharmaceutical composition is administered to a patient as a daily dose, once every 24 hrs.

In some embodiments, female sexual dysfunction is characterized by low libido or total lack of libido. In some particular embodiments, sexual dysfunction is characterized by a reduced or absent sexual desire or drive. In the preferred embodiments, sexual dysfunction is hypoactive libido.

In some embodiments, female sexual dysfunction is orgasmic dysfunction.

In some embodiments, sexual dysfunction is characterized by sexual aversion and lack of sexual pleasure.

In some embodiments, sexual dysfunction is vaginismus, dyspareunia.

In some embodiments, the pharmaceutical composition according to the invention is to be used in a non-therapeutic method for stimulating sexual activity in a subject in need thereof to improve and correct a woman's intimate relations and sex life, in the absence of the above diagnoses.

The invention also includes other types of sexual dysfunction not caused by organic disorders or diseases (somatic or psychiatric).

In some embodiments, the pharmaceutical composition is administered to a patient intranasally.

In particular embodiments, the pharmaceutical composition is administered as a spray.

In particular embodiments, a patient is a human.

### Brief Description of the drawings

**Fig. 1** Synthesis of pentapeptide Thr-Lys-Pro-Arg-Pro
**Fig. 2** Measurement results of the female sexual function index; the effect is expressed as a percentage from the baseline (before taking the medication). Column numbers correspond to the cohort numbers:
   1 - cohort #1, dose: 0.84 mg/day;
   2 - cohort #2, dose: 1.68 mg/day;
   3 - cohort #3, dose: 2.52 mg/day.

### Definitions and Terms

As used herein, the term "Libicor" refers to a pharmaceutical composition that is an aqueous solution comprising the following components at following concentrations, g/L:
Pentapeptide acetate Thr-Lys-Pro-Arg-Pro - 2- 20;
Benzalkonium chloride - 0.095-0.105;
Distilled water - remaining.

**Female sexual dysfunction:** impaired sexual response during arousal, main phase, orgasm, or resolution, pain during intercourse. The disorder, which includes both subjective and objective factors, prevents obtaining pleasure. In addition, it sometimes leads to infertility or otherwise related to it.

**Libido** (Latin: libido - attraction, desire, passion, drive) means sexual desire or sex drive. The mental component of sexual desire.

**Hypoactive libido, anaphrodisia** - lack or loss of sexual desire. Hypoactive libido is one of several sexual dysfunctions not caused by organic disorders or diseases (code F52.0 ICD-10).

**HSDD (Hypoactive sexual desire disorder)** - a disorder of decreased sexual desire.

**FSAD (Female sexual arousal disorder)** - a disorder of sexual arousal in women.

**FSIAD (Female sexual interest/arousal disorder)** -disorder of interest in sex/arousal in women.

The term **"therapeutically effective dose**" means such amount of the peptide that, when administered as part of mono- or combination therapy, produces a therapeutic effect sufficient for the treatment of female sexual dysfunction. The exact amount may vary from one individual to another, depending on the overall condition of the patient, severity of the condition, route of administration, treatment in combination with other drugs, etc. In some embodiments, the composition, which is the subject of the present invention, may be administered over the course of up to three months; in some other embodiments, over the course of one up to two months, in some particular embodiments over the course of up to one month. The preparation can be administered either once or several times a day, week (or any other time period) or from time to time. Moreover, the compounds can be administered to a patient daily over a certain time period (for example, 2-30 days), followed by a rest period, with no substance being administered (for example 1-30 days).

The invention relates to pharmaceutical compositions comprising the peptide according to the invention and may further comprise at least one pharmaceutically acceptable **excipient,** in particular, a carrier, a preservative, and/or a solvent, which can be administered to a patient together with the peptide that is the subject of the invention, and which doesn't impair the biological activity of said peptide and is not toxic when administered in the doses sufficient for the delivery of the effective dose of the peptide.

### Detailed description of the invention

The pharmaceutical compositions of the invention, and in particular the Libicor composition, can be effectively and safely administered to women one time or as a regimen for up to one month, with daily doses ranging from 840 mg to 5,040 mg. The pharmaceutical composition of the invention affects the brain structures responsible for the regulation of female sexual functions. The most effective delivery of the preparation into the brain occurs via the intranasal route because in any other mode of administration, the peptidases quickly degrade the preparation. From the nasal cavity, the preparation diffuses into the brain along the olfactory nerve fibers. The majority of olfactory bulbs are located on the olfactory epithelium, which is located on top of the nasal cavity in its middle part. In the preferred embodiments, the pharmaceutical composition is administered by a directional nasal spray.

### Embodiments

An objective observation of the achievement of the technical result upon administration of the invention was confirmed by statistically significant data demonstrated by the examples that contain experimental data. However, it should be understood that the invention is not limited to these and all the other examples set forth herein, and they are presented in this document with the sole purpose of illustrating the present invention.

### Synthesis of Thr-Lys-Pro-Arg-Pro pentapeptide

Synthesis of Thr-Lys-Pro-Arg-Pro pentapeptide was conducted using modern protecting groups and techniques to create a peptide bond in a solution. To form a peptide bond, the TBA salts method, the activated ester method, and the carbodiimide method were used. Both the step-by-step buildup of the peptide chain and the block method were used.

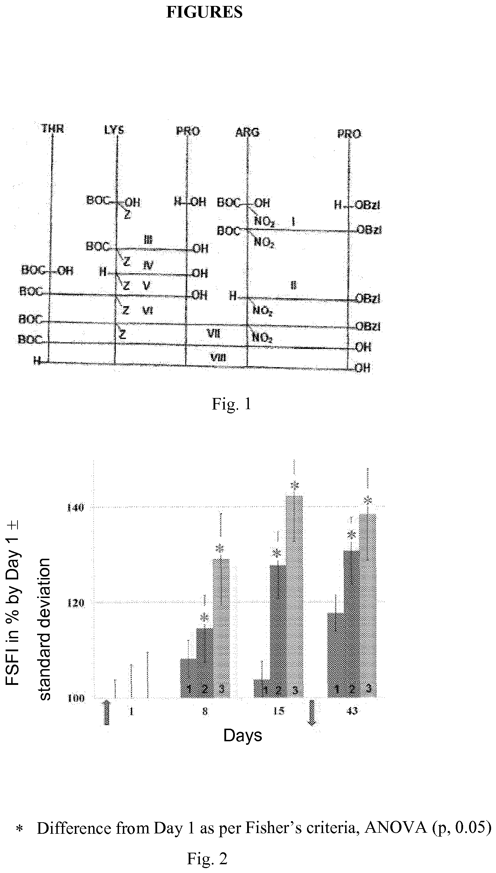

The synthesis of Thr-Lys-Pro-Arg-Pro pentapeptide was conducted as shown in Fig.1; the methods for the synthesis of said peptide are disclosed in detail in patent RU2507212.

### Preparation of the compositions of the invention

The pharmaceutical compositions for intranasal administration to a patient according to the invention can be prepared, in particular, in the procedure described below.

Purified water (for injection) was used for the preparation of the pharmaceutical composition according to the invention. Benzalkonium chloride was weighed and added to the purified water in the amount required to obtain 0.01% (w/w) solution of benzalkonium chloride. The solution was thoroughly mixed with a magnetic stirrer. The obtained solution was weighed. The necessary amount of the lyophilized active pharmaceutical ingredient pentapeptide acetate Thr-Lys-Pro-Arg-Pro was then weighed. The lyophilized active pharmaceutical ingredient pentapeptide acetate Thr-Lys-Pro-Arg-Pro was added to the solution of benzalkonium chloride in the amount required to obtain 0.2% - 1.8% (w/w) of the pentapeptide acetate Thr-Lys-Pro-Arg-Pro solution. The solution was thoroughly mixed with a magnetic stirrer. To reduce the bioburden, the obtained solution was filtered through a membrane filter with 0.2 - 0.22 mcm pores.

The obtained pharmaceutical composition was packaged, for example, into vials. For that purpose, dark brown vials, manufactured from the 1^{st} class hydrolytic glass, with a nominal volume of 10 ml, were washed and dried/sterilized separately. Maintaining the microbiological purity, each vial was placed on the scale and filled with 8.5 g of the prepared aqueous solution of benzalkonium chloride and pentapeptide Thr-Lys-Pro-Arg-Pro using a peristaltic pump. Maintaining the microbiological purity, each vial was capped with a snap-on nasal spray pump, 140 mcl in volume, CPS type (containing a microbiological filter for the incoming air), manufactured by Aptar Company, Germany. In some embodiments, when using CPS-type caps, the vials can be filled with pharmaceutical compositions not containing a preservative. The vials were closed manually using a mechanical closing device.

A label containing the information about the medication was then adhered to the vial. The obtained final product was stored at +2° - +4°C.

### Study of efficacy of the pharmaceutical compositions according to the invention as stimulants of sexual activity and sexual function.

The study described below was conducted to confirm the efficacy of the pharmaceutical composition of the invention and the treatment method.

### Study #1

A group of 15 physically healthy women aged 18 to 55 with low sex drive, which was diagnosed prior to the administration of the composition of the invention by asking them to fill out a questionnaire, were intranasally administered the composition of the invention at a pentapeptide concentration 2 g/L (Libicor) for two weeks. The women were divided into three cohorts of 5 people. Each cohort received different daily doses of Libicor from 840 mg to 2,520 mg. Prior to the administration of the medication, the women were tested to determine the level of sexual activity using the sexological questionnaire FEMALE SEXUAL FUNCTION INDEX (FSFI)©. After taking the medication, the women were tested again to record the effect. Testing was conducted a week after the start of Libicor, two weeks after, and a month after the cessation of Libicor administration. The standard safety profile, blood chemistry, complete blood count, urine analysis, sex hormone analysis, ECG, physical examination, and vital signs were also taken. The obtained results of this study are shown in Fig.1.

According to Fig.1, cohorts 2 and 3 show significant improvements over the baseline already one week into the therapy, which remain in effect one month after the cessation of the treatment. A dose-related effect was also observed. A 2,520 mcg/day dose showed high efficacy. The drug was well tolerated and safe. No adverse side effects were reported.

### Study #2

A group of 10 women were taking Libicor at a 9g/L concentration of pentapeptide intranasally for four weeks. At said concentration, a high compliance level was reported (i.e. using the medication once a day, one spray per each nostril), which is very important in this case, because the medication has to be refrigerated, and its use several times a day is quite inconvenient, which, as was shown in practice, leads to incompliance taking the medication. The women were receiving a 2,5 20-mg daily dose of Libicor. One group (5 people) used drip administration with a safety nose dropper. The second group (5 people) used a spray. Prior to the administration of the medication, the women were tested to determine the level of sexual activity using the sexological questionnaire FEMALE SEXUAL FUNCTION INDEX (FSFI)©. After taking the medication, the women were tested again to record the effect. Testing was conducted a week after the start of Libicor, two weeks after, four weeks after, and a month after the cessation of Libicor administration. The obtained results are presented in Table 1.

**Table 1. Comparative study results of various pharmaceutical compositions of the invention.**

| **Groups of women** | **Drug administrati on form** | **Dose** | **Average score before administra tion** | **Average score after one week of administra tion** | **Average score after two weeks of administra tion** | **Average score after four weeks of administra tion** | **Average score one month after cessation of administra tion** |
|---|---|---|---|---|---|---|---|
| **1^{st} group** | Drops, 0.9% solution | 2520 mg/day | 19 | 22 | 24 | 25 | 23 |
| **2^{nd} group** | Spray, 0.9% solution | 2520 mg/day | 18 | 23 | 26 | 27 | 25 |

The data presented in Table 1 demonstrate that the rate of the onset of the effect directly correlates with the administration route of the medication. Thus, the onset of the effect occurs somewhat faster and the effect itself is greater when using the spray than when using the drops, because when using the drops, some of the solution ends up in the nasal cavity and from there, in the esophagus due to the large size of the droplets, and thus, it isn't absorbed into the mucosa of the nasal cavity. Moreover, the distribution of liquid throughout the nasal cavity is considerably greater when the drop administration route is used, which also reduces the amount of the medication reaching the olfactory bulbs.

Noteworthy, that in terms of the interpretation of the questionnaire results, an improvement of 1 point is considered to be clinically significant. In this example, after four weeks of treatment with the pharmaceutical composition according to the invention, the scores improved by 6 and 9 points for the 1^{st} and 2^{nd} groups of women respectively, which indicated that the composition was highly effective. Moreover, the highest possible score according to the questionnaire is 36 points, which also confirms the powerful effect achieved after taking the composition of the invention, because the improvement occurred compared to the higher than average baseline, and thus, there was no effect of low baseline.

### Study of distribution of the medication according to the invention in the human body after intranasal administration

The study to determine how the medication of the invention is distributed through the body and reaches the brain was conducted on rats. Specially prepared rats were intranasally administered the pharmaceutical composition containing the pentapeptide of the invention radioactively-labelled with tritium. The animals were then euthanized at certain time intervals to be able to assess the biodistribution of the peptide in the brain and its transportation path. The study results demonstrated that after intranasal administration, a significant part of the medication reached the brain undegraded and was detected in the olfactory bulbs and other parts of the brain. Said results demonstrate that the olfactory epithelium serves as the main zone of entry for the drug into the brain, and that the olfactory nerve fibers are the transportation path.

The intranasal administration route, therefore, for the pharmaceutical composition of the invention, and in particular, using a spray, is the preferred administration route, which allows the medication to be directionally delivered into the olfactory epithelium region.

Although the invention has been described in connection with the disclosed embodiments, it will be obvious to a person skilled in the art that the specific experiments described in detail herein are for the sole purpose of illustrating the present invention and should not be construed as in any way limiting the scope of the invention. It should be understood that various modifications may be made without departing from the scope of the present invention.

## Claims

1. A liquid pharmaceutical composition suitable for intranasal administration comprising 2-20 g/l of a pentapeptide Thr-Lys-Pro-Arg-Pro (SEQ ID NO: 5) or a pharmaceutically acceptable salt thereof, and at least one pharmaceutically acceptable excipient, for use intranasally in the prevention and/or treatment of female sexual dysfunction in a subject, preferably a human.

2. The pharmaceutical composition for use according to claim 1, wherein the excipient is a carrier and/or a solvent, optionally further comprising a preservative, preferably wherein the preservative is benzalkonium chloride.

3. The pharmaceutical composition for use according to any of claims 1 or 2 comprising:
| | |
|---|---|
| pentapeptide Thr-Lys-Pro-Arg-Pro (SEQ ID NO: 5) or a pharmaceutically acceptable salt thereof | 2 - 20 g/l; |
| preservative | 0.095 - 0.105 g/l; |
| water | remaining. |

4. The pharmaceutical composition for use according to any of claims 1 to 3, wherein the pharmaceutically acceptable salt of pentapeptide Thr-Lys-Pro-Arg-Pro (SEQ ID NO: 5) is acetate, hydrochloride, phosphate, sulfate, mesylate, or tosylate.

5. The pharmaceutical composition for use according to any of claims 1-4, which is formulated as a solution.

6. The pharmaceutical composition for use according to claim 5, which is formulated as a spray.

7. The composition for use according to any of claims 1 to 6 for use in treating hypoactive sexual desire disorder (HSDD), female sexual arousal disorder (FSAD), or female sexual interest/arousal disorder (FSIAD).

8. The composition for use according to any of claims 1 to 7, wherein a daily dose of the pentapeptide Thr-Lys-Pro-Arg-Pro (SEQ ID NO: 5) or a pharmaceutically acceptable salt thereof is 800 - 5,100 mg, preferably wherein the daily dose of the peptide Thr-Lys-Pro-Arg-Pro (SEQ ID NO: 5) or a pharmaceutically acceptable salt thereof is selected from the group consisting of 840 mg, 1,680 mg, 2,520 mg, 3,360 mg, 4,200 mg and 5,040 mg.

9. The composition for use according to any of claims 1 to 8, wherein the pharmaceutical composition is administered daily for 1-31 days, preferably once a day.

10. The composition for use according to any of claims 1 to 9, wherein the female sexual dysfunction i) is **characterized by** a decreased libido or a total absence of libido; ii) is an orgasmic dysfunction; iii) is **characterized by** an aversion to sexual relations and lack of sexual pleasure, or iv) is vaginismus, dyspareunia, or hypoactive libido.

11. A non-therapeutic method for stimulating sexual activity in a subject in need thereof, said method comprising intranasally administering to the subject a liquid composition comprising 2-20 g/l of a pentapeptide Thr-Lys-Pro-Arg-Pro (SEQ ID NO: 5) or a pharmaceutically acceptable salt thereof and at least one pharmaceutically acceptable excipient.

12. The non-therapeutic method of claim 11, wherein the composition is in the form of a spray.

13. The non-therapeutic method of claim 11 or 12, for improving and correcting a woman's intimate relations and sex life.

## Patentansprüche

1. Flüssige pharmazeutische Zusammensetzung, die zur intranasalen Verabreichung geeignet ist, umfassend 2-20 g/l eines Pentapeptids Thr-Lys-Pro-Arg-Pro (SEQ ID NO: 5) oder eines pharmazeutisch verträglichen Salzes davon und mindestens einen pharmazeutisch verträglichen Hilfsstoff, zur intranasalen Verwendung bei der Prävention und/oder Behandlung von weiblicher sexueller Dysfunktion bei einem Subjekt, vorzugsweise einem Menschen.

2. Pharmazeutische Zusammensetzung zur Verwendung nach Anspruch 1, wobei der Hilfsstoff ein Träger und/oder ein Lösungsmittel ist, gegebenenfalls ferner umfassend ein Konservierungsmittel, wobei das Konservierungsmittel vorzugsweise Benzalkoniumchlorid ist.

3. Pharmazeutische Zusammensetzung zur Verwendung nach einem der Ansprüche 1 oder 2, umfassend:
| | |
|---|---|
| Pentapeptid Thr-Lys-Pro-Arg-Pro (SEQ ID NO: 5) oder ein pharmazeutisch verträgliches Salz davon | 2-20 g/l; |
| Konservierungsmittel | 0,095 - 0,105 g/l; |
| Wasser | der Rest. |

4. Pharmazeutische Zusammensetzung zur Verwendung nach einem der Ansprüche 1 bis 3, wobei das pharmazeutisch verträgliche Salz des Pentapeptids Thr-Lys-Pro-Arg-Pro (SEQ ID NO: 5) Acetat, Hydrochlorid, Phosphat, Sulfat, Mesylat oder Tosylat ist.

5. Pharmazeutische Zusammensetzung zur Verwendung nach einem der Ansprüche 1-4, die als Lösung formuliert ist.

6. Pharmazeutische Zusammensetzung zur Verwendung nach Anspruch 5, die als Spray formuliert ist.

7. Zusammensetzung zur Verwendung nach einem der Ansprüche 1 bis 6 zur Verwendung bei der Behandlung einer hypoaktiven Störung des sexuellen Verlangens (HSDD), einer Störung der weiblichen sexuellen Erregung (FSAD) oder einer Störung des weiblichen sexuellen Interesses/der Erregung (FSIAD).

8. Zusammensetzung zur Verwendung nach einem der Ansprüche 1 bis 7, wobei eine Tagesdosis des Pentapeptids Thr-Lys-Pro-Arg-Pro (SEQ ID NO: 5) oder eines pharmazeutisch verträglichen Salzes davon 800 - 5.100 mg beträgt, wobei vorzugsweise die Tagesdosis des Peptids Thr-Lys-Pro-Arg-Pro (SEQ ID NO: 5) oder eines pharmazeutisch verträglichen Salzes davon ausgewählt ist aus der Gruppe bestehend aus 840 mg, 1.680 mg, 2.520 mg, 3.360 mg, 4.200 mg und 5.040 mg.

9. Zusammensetzung zur Verwendung nach einem der Ansprüche 1 bis 8, wobei die pharmazeutische Zusammensetzung 1-31 Tage lang täglich, vorzugsweise einmal täglich, verabreicht wird.

10. Zusammensetzung zur Verwendung nach einem der Ansprüche 1 bis 9, wobei die weibliche sexuelle Dysfunktion i) durch eine verminderte Libido oder eine völlige Abwesenheit von Libido gekennzeichnet ist; ii) eine orgasmische Dysfunktion ist; iii) durch eine Abneigung gegen sexuelle Beziehungen und einen Mangel an sexuellem Vergnügen gekennzeichnet ist, oder iv) Vaginismus, Dyspareunie oder hypoaktive Libido ist.

11. Nicht-therapeutisches Verfahren zur Stimulierung der sexuellen Aktivität bei einem Patienten, der diese benötigt, wobei das Verfahren die intranasale Verabreichung einer flüssigen Zusammensetzung enthaltend 2-20 g/l eines Pentapeptids Thr-Lys-Pro-Arg-Pro (SEQ ID NO: 5) oder eines pharmazeutisch verträglichen Salzes davon und mindestens einen pharmazeutisch verträglichen Hilfsstoff umfasst.

12. Nicht-therapeutisches Verfahren nach Anspruch 11, wobei die Zusammensetzung in Form eines Sprays vorliegt.

13. Nicht-therapeutisches Verfahren nach Anspruch 11 oder 12 zur Verbesserung und Korrektur der intimen Beziehungen und des Sexuallebens einer Frau.

## Revendications

1. Composition pharmaceutique liquide convenant pour une administration par voie intranasale, comprenant 2 à 20 g/l d'un pentapeptide Thr-Lys-Pro-Arg-Pro (SEQ ID NO : 5) ou d'un sel pharmaceutiquement acceptable de celui-ci, et au moins un excipient pharmaceutiquement acceptable, pour une utilisation par voie intranasale dans la prévention et/ou le traitement d'un dysfonctionnement sexuel féminin chez un sujet, de préférence un humain.

2. Composition pharmaceutique pour une utilisation selon la revendication 1, dans laquelle l'excipient est un véhicule et/ou un solvant, comprenant éventuellement en outre un conservateur, de préférence dans laquelle le conservateur est le chlorure de benzalkonium.

3. Composition pharmaceutique pour une utilisation selon l'une quelconque des revendications 1 ou2, comprenant :
| | |
|---|---|
| pentapeptide Thr-Lys-Pro-Arg-Pro (SEQ ID NO : 5) ou un sel pharmaceutiquement acceptable de celui-ci | 2 à 20 g/l ; |
| conservateur | 0,095 à 0,105 g/l |
| eau | le reste. |

4. Composition pharmaceutique pour une utilisation selon l'une quelconque des revendications 1 à 3, dans laquelle le sel pharmaceutiquement acceptable du pentapeptide Thr-Lys-Pro-Arg-Pro (SEQ ID NO : 5) est un acétate, chlorhydrate, phosphate, sulfate, mésylate, ou tosylate.

5. Composition pharmaceutique pour une utilisation selon l'une quelconque des revendications 1 à 4, qui est formulée en une solution.

6. Composition pharmaceutique pour une utilisation selon la revendication 5, qui est formulée en un spray.

7. Composition pharmaceutique pour une utilisation selon l'une quelconque des revendications 1 à 6, pour une utilisation dans le traitement d'un trouble du désir sexuel hypoactif (HSDD), d'un trouble de l'excitation sexuelle chez la femme (FSAD), ou d'un trouble de l'intérêt/excitation sexuelle chez la femme (FSIAD).

8. Composition pharmaceutique pour une utilisation selon l'une quelconque des revendications 1 à 7, dans laquelle une dose quotidienne du pentapeptide Thr-Lys-Pro-Arg-Pro (SEQ ID NO : 5) ou d'un sel pharmaceutiquement acceptable de celui-ci est de 800 à 5100 mg, de préférence dans laquelle la dose quotidienne du peptide Thr-Lys-Pro-Arg-Pro (SEQ ID NO : 5) ou d'un sel pharmaceutiquement acceptable de celui-ci est choisie dans le groupe constitué par 840 mg, 1680 mg, 2520 mg, 3360 mg, 4200 mg et 5040 mg.

9. Composition pharmaceutique pour une utilisation selon l'une quelconque des revendications 1 à 8, laquelle composition pharmaceutique est administrée chaque jour pendant 1 à 31 jours, de préférence une fois par jour.

10. Composition pharmaceutique pour une utilisation selon l'une quelconque des revendications 1 à 9, dans laquelle le dysfonctionnement sexuel féminin i) est **caractérisé par** une libido réduite ou une absence totale de libido ; ii) est un dysfonctionnement orgasmique ; iii) est **caractérisé par** une aversion pour les relations sexuelles et un manque de plaisir sexuel, ou iv) est un vaginisme, une dyspareunie, ou une libido hypoactive.

11. Méthode non thérapeutique pour stimuler l'activité sexuelle chez un sujet en ayant besoin, ladite méthode comprenant l'administration par voie intranasale au sujet d'une composition liquide comprenant 2 à 20 g/l d'un pentapeptide Thr-Lys-Pro-Arg-Pro (SEQ ID NO : 5) ou d'un sel pharmaceutiquement acceptable de celui-ci et au moins un excipient pharmaceutiquement acceptable.

12. Méthode non thérapeutique selon la revendication 11, dans laquelle la composition est sous la forme d'un spray.

13. Méthode non thérapeutique selon la revendication 11 ou 12, pour améliorer et corriger les relations intimes et la vie sexuelle d'une femme.
